# EUROPEAN PATENT SPECIFICATION

(11) **EP 1 721 955 B1**
(45) Date of publication and mention of the grant of the patent: **03.08.2011**
(21) Application number: 06113552.1
(22) Date of filing: 05.05.2006
(51) Int. Cl.: C10L 1/232, C10L 1/188, C07D 213/18

(54) **Fuel compositions**
Kraftstoffzusammensetzungen
Compositions de carburant

(30) Priority: 11.05.2005 EP 05270015
(43) Date of publication of application: 15.11.2006
(73) Proprietor: Infineum International Limited, Abingdon, Oxfordshire OX13 6BB (GB)
(72) Inventor: Breakspear, Angela Priscilla, Abingdon Oxfordshire OX13 6BB (GB); Caprotti, Rinaldo, Abingdon Oxfordshire OX13 6BB (GB); Fava, Carlo S., Abingdon Oxfordshire OX13 6BB (GB)
(74) Representative: Capaldi, Michael Joseph

(56) References cited:
- EP-A- 0 385 778
- WO-A2-01/79397
- US-B1- 6 328 771
- HIROSE, KATSUMI ET AL: "Reaction of pyridine bases with carboxylic acids in benzene" BULLETIN OF THE CHEMICAL SOCIETY OF JAPAN , 50(3), 608-13 CODEN: BCSJA8; ISSN: 0009-2673, 1977, XP002383673

## Description

This invention relates to middle distillate fuels such as diesel fuels, kerosene and jet fuel.

Environmental concerns have led to a need for fuels with reduced sulphur content, especially diesel fuel, heating oil and kerosene. However, the refining processes that produce fuels with low sulphur contents also result in a product of lower viscosity and a lower content of other components in the fuel that contribute to its lubricity, for example, polycyclic aromatics and polar compounds. Furthermore, sulphur-containing compounds in general are regarded as providing some anti-wear properties and a result of the reduction in their proportions, together with the reduction in proportions of other components providing lubricity, has been an increase in the number of reported problems in fuel pumps in diesel engines. The problems are caused by wear in, for example, cam plates, rollers, spindles and drive shafts, which may result in sudden pump failures relatively early in the life of the engine.

The problems may be expected to become worse in future because, in order to meet stricter requirements on exhaust emissions generally, higher pressure fuel systems, including in-line, rotary pumps, common-rail pumps and unit injector systems, are being introduced, these being expected to have more stringent lubricity requirements than present equipment, at the same time as lower sulphur levels in fuels become more widely required.

Historically, the typical sulphur content in a diesel fuel was below 0.5% by weight. In Europe maximum sulphur levels have been reduced from 0.20% to 0.05% and in Sweden grades of fuel with levels below 0.005% (Class 2) and 0.001% (Class 1) are in use. A fuel oil composition with a sulphur level below 0.05% by weight is referred to herein as a low-sulphur fuel.

Such low-sulphur fuels may contain an additive to enhance their lubricity. These additives are of several types. In WO 94/17160, there is disclosed a low sulphur fuel comprising a carboxylic acid ester to enhance lubricity, more especially an ester in which the acid moiety contains from 2 to 50 carbon atoms and the alcohol moiety contains one or more carbon atoms. In U.S. Patent No. 3273981, a mixture of a dimer acid, for example, the dimer of linoleic acid, and a partially esterified polyhydric alcohol is described for the same purpose. In U.S. Patent No. 3287273, the use of an optionally hydrogenated dimer acid glycol ester is described. Other materials used as lubricity enhancers, or anti-wear agents as they are also termed, include a sulphurized dioleyl norbornene ester (EP-A-99595), castor oil (U.S. Patent No. 4375360 and EP-A-605857) and, in methanol-containing fuels, a variety of alcohols and acids having from 6 to 30 carbon atoms, acid and alcohol ethoxylates, mono-and di-esters, polyol esters, and olefin-carboxylic acid copolymers and vinyl alcohol polymers (also U.S. Patent No. 4375360).

Such additives are commonly provided in the form of additive compositions where the active species is dissolved in a solvent, optionally together with other additives. Although largely effective as lubricity additives, it has been found that certain carboxylic acids and carboxylic acid esters, as well as other known lubricity additives, have the disadvantage of poor solubility at low temperature. This poor solubility is found in relation to the solvents used to provide additive compositions (normally hydrocarbon based), and also in relation to the fuels into which the additive is added. This places a limitation on the storage and use of certain known lubricity additives at low ambient temperatures.

US 6,328,771 describes the use of the product of the reaction between a carboxylic acid and a heterocyclic aromatic amine to improve the lubricity of a low sulphur content fuel oil. Examples of heterocyclic aromatic amines include pyridine, pyrazine, pyrolle, pyrazole and imidazole, with imidazole being particularly preferred.

EP 0 798 364 A1 describes the use of a salt formed by the reaction between a carboxylic acid and an aliphatic amine to improve *inter alia,* the lubricity of a diesel fuel.

Katasumi Hirose et al., in the "Bulletin of the Chemical Society of Japan", 50(3), (1977), pp 608-613, describe the reaction of various pyridine bases with various carboxylic acids and derive certain thermodynamic and kinetic parameters.

The present invention is based on salts derived from hydrocarbyl derivatives of pyridine, and the observation that such salts, when added to a middle distillate fuel oil, improve the lubricity thereof. It is a further advantage that the salts of the invention have greatly improved low temperature solubility when compared to more conventional lubricity enhancers. It is also an advantage that the salts of the invention have relatively high flash points, compared to for example, the salts described in US 6,328,771. This leads to benefits in terms of the ease of, and hazards associated with, their handling and transportation.

Thus in accordance with a first aspect, the present invention provides a middle distillate fuel oil composition comprising a major amount of a fuel oil and a minor amount of a salt formed by the reaction of a carboxylic acid with a hydrocarbyl derivative of pyridine.

In this specification, the use of the term 'salt' to describe the product formed by the reaction of the carboxylic acid and the derivative of pyridine should not be taken to mean that the reaction necessarily forms a pure salt. It is presently believed that the reaction does form a salt and thus that the reaction product contains such as salt however, due to the complexity of the reaction, it is likely that other species will also be present. The term 'salt' should thus be taken to include not only the pure salt species, but also the mixture of species formed during the reaction of the carboxylic acid and the hydrocarbyl derivative of pyridine.

Preferably, the hydrocarbyl derivative of pyridine is represented by the formula: 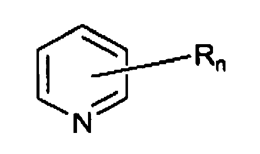 wherein n is an integer between 1 and 5; and wherein each R independently represents a hydrocarbyl group having between 1 and 30 carbon atoms.

Preferably, n is at least 2.

Preferably, each R independently represents a hydrocarbyl group having between 1 and 15 carbon atoms, more preferably, between 1 and 8 carbon atoms.

As used in this specification, the term 'hydrocarbyl' refers to a group having a carbon atom directly attached to the rest of the molecule and having a hydrocarbon or predominantly hydrocarbon character. Among these, there may be mentioned aliphatic groups such as alkyl and alkenyl groups. Such groups may be saturated or unsaturated, linear or branched. The groups may also contain non-hydrocarbon substituents provided their presence does not alter the predominantly hydrocarbon character of the group. Examples include keto, halo, hydroxy, nitro, cyano, alkoxy and acyl. If the hydrocarbon group is substituted, a single (mono) substituent is preferred. The groups may also or alternatively contain atoms other that carbon in a chain otherwise composed of carbon atoms. Suitable hetero atoms include for example, nitrogen, oxygen or sulphur.

Preferably, at least one group R represents an alkyl or alkenyl group. The following structures are representative of the types of species contemplated as hydrocarbyl derivatives of pyridine. These are included merely to illustrate the present invention and are not to be considered limiting in any way.

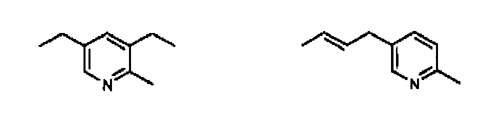

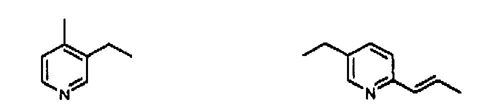

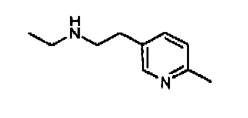

The hydrocarbyl derivative of pyridine may be used as a single species or as a mixture of two or more different species in any ratio. A suitable commercial source of hydrocarbyl derivatives of pyridine are 'Alkolidine' products available from Lonza Ltd.

As carboxylic acid, those corresponding to the formula [R'(COOH)ₓ]_{y}, where each R' is independently a hydrocarbon group of between 2 and 30 carbon atoms, and x is an integer between 1 and 4, are suitable. Preferably, R' is a hydrocarbon group of 8 to 24 carbon atoms, more preferably, 12 to 20 carbon atoms. Preferably, x is 1 or 2, more preferably, x is 1. Preferably, y is 1, in which case the acid has a single R' group. Alternatively, the acid may be a dimer, trimer or higher oligomer acid, in which case y will be greater than 1 for example 2, 3 or 4 or more. R' is suitably an alkyl or alkenyl group which may be linear or branched. Examples of carboxylic acids which may be used in the present invention include: lauric acid, myristic acid, palmitic acid, stearic acid, isostearic acid, neodecanoic acid, arachic acid, behenic acid, lignoceric acid, cerotic acid, montanic acid, melissic acid, caproleic acid, oleic acid, elaidic acid, linoleic acid, linolenic acid, coconut oil fatty acid, soy bean fatty acid, tall oil fatty acid, fish oil fatty acid, rapeseed oil fatty acid, tallow oil fatty acid and palm oil fatty acid. Mixtures of two or more acids in any proportion are also suitable. Also suitable are the anhydrides of carboxylic acids and mixtures thereof. In a preferred embodiment, the carboxylic acid comprises tall oil fatty acid (TOFA). It has been found that TOFA with a saturate content of less than 5% by weight is especially suitable. Preferably, TOFA with a abietic acid content of less than 5% by weight, for example, less than 2% by weight, is used.

In another preferred embodiment, the carboxylic acid comprises soy bean fatty acid (SOFA).

Also suitable are aromatic carboxylic acids and their alkyl derivatives as well as aromatic hydroxy acids and their alkyl derivatives. Illustrative examples include benzoic acid, salicylic acid and acids derived from such species.

Preferably, the carboxylic acid has an iodine value of at least 100 g/100g, more preferably at least 130 g/100g, for example, at least 150 g/100g. This has been found to further improve the low temperature solubility of the salts of the invention.

Preferably, the salt has a flash point of at least 50°C, more preferably at least 60°C.

The salt may conveniently be produced by mixing the carboxylic acid with the hydrocarbyl derivative of pyridine. The order in which one component is added to the other is not important. The molar ratio of the amount of acid to the amount of hydrocarbyl derivative of pyridine is preferably from 10:1 to 1:10, more preferably from 10:1 to 1:2, for example, around 1:1. The reaction may be conducted at room temperature, but is preferably heated gently, for example to 40°C.

The fuel oil is a middle distillate fuel oil. Such distillate fuel oils generally boil within the range of from 110°C to 500°C, e.g. 150°C to 400°C. The fuel oil may comprise atmospheric distillate or vacuum distillate, cracked gas oil, or a blend in any proportion of straight run and thermally and/or refinery streams such as catalytically cracked and hydro-cracked distillates. The most common petroleum distillate fuels are kerosene, jet fuels, diesel fuels, heating oils and heavy fuel oils. The heating oil may be a straight atmospheric distillate, or it may contain minor amounts, e.g. up to 35 wt %, of vacuum gas oil or cracked gas oil or of both.

Other examples of fuel oils include Fischer-Tropsch fuels. Fischer-Tropsch fuels, also known as FT fuels, include those described as gas-to-liquid (GTL) fuels, biomass-to-liquid (BTL) fuels and coal conversion fuels. To make such fuels, syngas (CO + H₂) is first generated and then converted to normal paraffins by a Fischer-Tropsch process. The normal paraffins may then be modified by processes such as catalytic cracking/reforming or isomerisation, hydrocracking and hydroisomerisation to yield a variety of hydrocarbons such as iso-paraffins, cyclo-paraffins and aromatic compounds. The resulting FT fuel can be used as such or in combination with other fuel components and fuel types such as those mentioned in this specification.

Preferably, the fuel oil has a sulphur content of at most 0.2% by weight, more preferably of at most 0.05% by weight, especially of at most 0.035%, for example, at most 0.015%. Fuels with even lower levels of sulphur are also suitable such as, fuels with less than 50ppm sulphur by weight, preferably less than 20 ppm, for example 10ppm or less.

Preferably, the salt is present in the fuel oil at a level of between 5 and 1000 ppm by weight, more preferably between 10 and 500 ppm, even more preferably between 10 and 250 ppm, especially between 10 and 150 ppm, for example, between 50 and 150 ppm.

In accordance with a second aspect, the present invention provides the use of a salt as defined in relation to the first aspect, to improve the lubricity of a fuel oil having a sulphur content of less than 0.2% by weight.

The salt may be used singly or as a mixture of more than one salt. It may also be used in combination with one or more co-additives such as are known in the art, for example the following: detergents, antioxidants (to avoid fuel degradation), corrosion inhibitors, dehazers, demulsifiers, metal deactivators, antifoaming agents, cetane improvers, cosolvents, package compatibilisers, reodourants, additives to improve the regeneration of particulate traps, middle distillate cold flow improvers and other lubricity additives.

The invention will now be described by way of example only.

### Example 1

In this example, the hydrocarbyl derivative of pyridine used was Alkolidine 10 which is a product of Lonza Ltd and is a mixture of several hydrocarbyl derivatives of pyridine.

Alkolidine 10 (17.0g, 95 mmoles) was added to a beaker and heated to 40°C with stirring. Tall oil fatty acid, with a saturate content of ca. 2% and a rosin acid content of ca. 1%, (TOFA) (25.4g, 88 mmoles) was then added to the beaker. An exotherm of ca. 12°C was measured indicating that the two components reacted. FTIR analysis of the reaction product showed a reduction in the strong carboxylic acid peak at 1710cm⁻¹ compared to the starting acid, and a corresponding appearance of carboxylate peaks at 1560 and 1458 cm⁻¹ as well as the appearance of a broad peak at 1935cm⁻¹ assignable to a pyridinium species. This was a clear indication of the formation of a salt. The flash-point of the reaction product was 116°C.

### Example 2

Example 1 was repeated using Alkolidine 10 (17.0g, 95 mmoles) and tall oil fatty acid, with a saturate content of ca. 2% and a rosin acid content of ca. 2%, (TOFA) (25.4g, 88 mmoles). An exotherm of ca. 12°C was measured indicating that the two components reacted. FTIR analysis of the reaction product showed a reduction in the strong carboxylic acid peak at 1710cm⁻¹ compared to the starting acid, and a corresponding appearance of carboxylate peaks at 1560 and 1458cm⁻¹ as well as the appearance of a broad peak at 1935cm⁻¹ assignable to a pyridinium species. This was a clear indication of the formation of a salt. The flash-point of the reaction product was 116°C.

### Example 3 (Comparative)

Example 1 was repeated using pyridine (87.6g, 1107 mmoles) in place of Alkolidine 10 and the same TOFA (320g, 1107 mmoles). An exotherm was again detected indicating that the two components reacted. The flash-point of the reaction product was 46°C.

### Example 4 (Comparative)

Example 2 was repeated using pyridine (87.6g, 1107 mmoles) in place of Alkolidine 10 and the same TOFA (320g, 1107 mmoles). An exotherm was again detected indicating that the two components reacted. The flash-point of the reaction product was 46°C.

### HFRR testing

The salts prepared in Examples 1 and 2 above were tested in Mk 1 diesel (< 10 ppm S) using the High Frequency Reciprocating Rig (HFRR) test in accordance with BS EN ISO 12156-1 (2000). Results are given in Table 1 below together with results of similar tests using Comparative Examples 3 and 4.

**Table 1**

| | **Treat rate /ppm** | **Average wear scar /µm** | | **Treat rate / ppm** | **Average wear scar** / **µm** |
|---|---|---|---|---|---|
| **Base fuel** | 0 | 649 | **Base fuel** | 0 | 661 |
| **Example 1** | 50 | 668 | **Comparative Example 3** | 50 | 667 |
| | 100 | 499 | | 100 | 538 |
| | 150 | 470 | | 150 | 487 |
| | 200 | 406 | | 200 | 436 |
| | 250 | 420 | | 250 | 422 |
| | 300 | 446 | | 300 | 343 |
| **Example 2** | 50 | 675 | **Comparative Example 4** | 50 | 670 |
| | 100 | 611 | | 100 | 515 |
| | 150 | 511 | | 150 | 475 |
| | 200 | 467 | | 200 | 451 |
| | 250 | 424 | | 250 | 380 |
| | 300 | 404 | | 300 | 428 |

The results indicate that the lubricity performance of the salts of the invention is comparable to that found for simple pyridine salts such as those described in US 6,328,771.

### Stability Testing

Additive concentrates of the salts of Examples 1 and 2 were made up as 10, 33, 50 and 75% solutions in Solvesso 100 and tested for low temperature stability at -30°C and -40°C alongside similar solutions of Comparative Examples 3 and 4. The non-diluted salts were also tested (100% concentration). The results are given in Table 2 below. In the table, the data refer to the number of days that the test sample remained clear and bright. All tests were run for a maximum of 28 days, so a result of '28' in the Table does not indicate a failure on day 28.

**Table 2**

| **Additive concentration** | **Temperature /°C** | **Example 1** | **Example 2** | **Comparative Example 3** | **Comparative Example 4** |
|---|---|---|---|---|---|
| 10% | -30 | 28 | 28 | 3 | 28 |
| | -40 | 28 | 28 | 0 | 1 |
| 33% | -30 | 28 | 28 | 0 | 0 |
| | -40 | 7 | 28 | 0 | 0 |
| 50% | -30 | 28 | 28 | 0 | 0 |
| | -40 | 7 | 28 | 0 | 0 |
| 75% | -30 | 14 | 28 | 0 | 0 |
| | -40 | 2 | 28 | 0 | 0 |
| 100% | -30 | 6 | 28 | 0 | 0 |
| | -40 | 0 | 0 | 0 | 0 |

It is clear from the results given in Table 2 that the low temperature stability of the salts of the present invention far exceeds that found for simple pyridine salts such as those described in US 6,328,771.

## Claims

1. A middle distillate fuel oil composition comprising a major amount of a middle distillate fuel oil and a minor amount of a salt formed by the reaction of a carboxylic acid with a hydrocarbyl derivative of pyridine.

2. A middle distillate fuel oil composition according to claim 1, wherein the hydrocarbyl derivative of pyridine is represented by the formula: 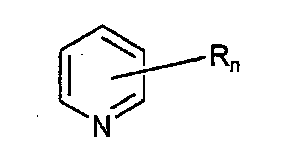 wherein n is an integer between 1 and 5; and wherein each R independently represents a hydrocarbyl group having between 1 and 30 carbon atoms.

3. A middle distillate fuel oil composition according to claim 2, wherein n is at least 2.

4. A middle distillate fuel oil composition according to claim 2 or claim 3, wherein at least one group R represents an alkyl or alkenyl group.

5. A middle distillate fuel oil composition according to any preceding claim wherein the carboxylic acid comprises a fatty acid or a mixture of fatty acids.

6. A middle distillate fuel oil composition according to any preceding claim wherein the carboxylic acid comprises tall oil fatty acid (TOFA).

7. A middle distillate fuel oil composition according to any preceding claim, wherein the middle distillate fuel oil has a sulphur content of less than 0.05% by weight.

8. The use of a salt as defined in any of claims 1 to 6 to improve the lubricity of a middle distillate fuel oil having a sulphur content of less than 0.2% by weight.

## Patentansprüche

1. Mitteldestillatbrennstofföl-Zusammensetzung, die eine größere Menge an Mitteldestillat-Brennstofföl und eine kleinere Menge Salz umfasst, das durch die Reaktion von Carbonsäure mit einem Kohlenwasserstoffderivat von Pyridin gebildet ist.

2. Mitteldestillat-Brennstoffölzusammensetzung nach Anspruch 1, wobei das Kohlenwasserstoffderivat von Pyridin durch die Formel 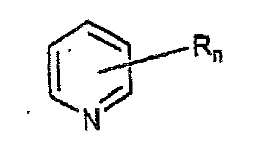 wiedergegeben wird, in der n eine ganze Zahl zwischen 1 und 5 ist und in der jedes R unabhängig eine Kohlenwasserstoffgruppe mit zwischen 1 und 30 Kohlenwasserstoffatomen wiedergibt.

3. Mitteldestillat-Brennstoffölzusammensetzung nach Anspruch 2, wobei n mindestens 2 ist.

4. Mitteldestillat-Brennstoffölzusammensetzung nach Anspruch 2 oder Anspruch 3, wobei mindestens eine Gruppe R eine Alkylgruppe oder Alkenylgruppe darstellt.

5. Mitteldestillat-Brennstoffölzusammensetzung nach einem der vorhergehenden Ansprüche, wobei die Carbonsäure eine Fettsäure oder eine Mischung von Fettsäuren umfasst.

6. Mitteldestillat-Brennstoffölzusammensetzung nach einem der vorhergehenden Ansprüche, wobei die Carbonsäure Talgölfettsäure (TOFA) umfasst.

7. Mitteldestillat-Brennstoffölzusammensetzung nach einem der vorhergehenden Ansprüche, wobei das Mitteldestillat-Brennstofföl einen Schwefelgehalt von weniger als 0.05 Gew.-% aufweist.

8. Verwendung eines Salzes wie in einem der Ansprüche 1 bis 6 definiert zur Verbesserung der Schmierfähigkeit eines Mitteldestillat-Brennstofföls, das einen Schwefelgehalt von weniger als 0,2 Gew.-% aufweist.

## Revendications

1. Composition de fuel oil distillé moyen comprenant une quantité dominante d'un fuel oil distillé moyen et une petite quantité d'un sel formé par réaction d'un acide carboxylique avec un dérivé hydrocarbylique de pyridine.

2. Composition de fuel oil suivant la revendication 1, dans laquelle le dérivé hydrocarbylique de pyridine est représenté par la formule 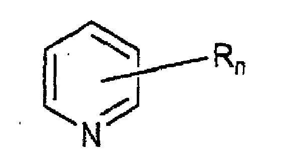 dans laquelle n représente un nombre entier de 1 à 5 ; et dans laquelle chaque groupe R représente indépendamment un groupe hydrocarbyle ayant 1 à 30 atomes de carbone.

3. Composition de fuel oil suivant la revendication 2, dans laquelle n est égal à au moins 2.

4. Composition de fuel oil suivant la revendication 2 ou la revendication 3, dans laquelle au moins un groupe R représente un groupe alkyle ou alcényle.

5. Composition de fuel oil suivant l'une quelconque des revendications précédentes, dans laquelle l'acide carboxylique comprend un acide gras ou un mésange d'acides gras.

6. Composition de fuel oil suivant l'une quelconque des revendications précédentes, dans laquelle l'acide carboxylique comprend l'acide gras du tallöl (TOFA).

7. Composition de fuel oil distillé moyen suivant l'une quelconque des revendications précédentes, dans laquelle ledit fuel oil distillé moyen a une teneur en soufre inférieure à 0,05 % en poids.

8. Utilisation d'un sel tel que défini dans l'une quelconque des revendications 1 à 6 pour améliorer le pouvoir lubrifiant d'un fuel oil distillé moyen ayant une teneur en soufre inférieure à 0,2 % en poids.
